# FASCICULE DE BREVET EUROPEEN

(11) **EP 2 683 297 B1**
(45) Date de publication et mention de la délivrance du brevet: **15.02.2017**
(21) Numéro de dépôt: 12712332.1
(22) Date de dépôt: 06.03.2012
(51) Int. Cl.: A61B 5/117, C07D 403/04, C07D 257/08, G01N 21/64

(54) **TROUSSE DE REVELATION COMPRENANT UN AGENT FLUORESCENT ET UN CYANOACRYLATE, PROCEDE DE CO-FUMIGATION D'UN AGENT FLUORESCENT ET D'UN CYANOACRYLATE**
FLUORESZIERENDES MITTEL UND CYANACRYLAT ENTHALTENDES NACHWEISKIT, VERFAHREN ZUM MITBEGASSEN EINES FLUORESZIERENDEN MITTEL UND EINES CYANOACRYLATES
VISUALIZATION KIT CONTAINING A FLUORESCENT AGENT AND A CYANOACRYLATE AND CO-FUMIGATION PROCESS OF A FLUORESCENT AGENT AND A CYANOACRYLATE

(30) Priorité: 07.03.2011 FR 1151828
(43) Date de publication de la demande: 15.01.2014
(73) Titulaire: Crime Science Technology, 59120 Loos (FR); Ecole Normale Superieure De Cachan, 94230 Cachan (FR); Centre National de la Recherche Scientifique (C.N.R.S.), 75016 Paris (FR)
(72) Inventeur: PRETE, Cosimo, F-59650 Villeneuve D'ascq (FR); AUDEBERT, Pierre, F-94235 Cachan Cédex (FR); GALMICHE, Laurent, F-94235 Cachan Cédex (FR); ALLAIN, Clémence, F-94235 Cachan Cédex (FR)
(74) Mandataire: Cabinet Plasseraud
(86) Numéro de dépôt international: PCT/FR2012/050462
(87) Numéro de publication internationale: WO 2012/120234

(56) Documents cités:
- WO-A2-2008/020951
- US-A- 4 504 408
- TANIUCHI ET AL: "ENZYMATIC FORMATION OF CATECHOL FROM ANTHRANILIC ACID.", JOURNAL OF BIOLOGICAL CHEMISTRY, vol. 239, 1 juillet 1964 (1964-07-01), pages 2204-2211, XP55027764, ISSN: 0021-9258
- THERALD MOELLER ET AL: "Analytical Applications of 8-Hydroxyquinoline Derivatives of Gallium and Thallium", ANALYTICAL CHEMISTRY, vol. 22, no. 5, 1 mai 1950 (1950-05-01), pages 686-690, XP55027760, ISSN: 0003-2700, DOI: 10.1021/ac60041a020
- GONG Y-H ET AL: "Synthesis and Physical Chemistry of s-Tetrazines: Which Ones are Fluorescent and Why?", EUROPEAN JOURNAL OF ORGANIC CHEMISTRY, WILEY-VCH, WEINHEIM; DE, vol. 2009, no. 35, 30 octobre 2009 (2009-10-30), pages 6121-6128, XP002633987, ISSN: 1434-193X, DOI: DOI:10.1002/EJOC.200900964
- GILLES CLAVIER ET AL: "s -Tetrazines as Building Blocks for New Functional Molecules and Molecular Materials", CHEMICAL REVIEWS, vol. 110, no. 6, 9 juin 2010 (2010-06-09), pages 3299-3314, XP055001208, ISSN: 0009-2665, DOI: 10.1021/cr900357e
- S. MORITOMO ET AL.: "A new method to enhance visualization of latent fingermarks by sublimating dyes...", FORENSIC SCIENCE INTERNATIONAL., vol. 97, 1998, pages 101-108, XP002644449, ELSEVIER SCIENTIFIC PUBLISHERS IRELAND LTD. ISSN: 0379-0738

## Description

L'invention concerne une trousse de révélation comprenant un agent fluorescent et un cyanoacrylate, un procédé de co-fumigation d'un agent fluorescent et d'un cyanoacrylate et l'utilisation de ce procédé pour la révélation de traces, notamment de traces papillaires.

Les traces papillaires sont un des outils les plus efficaces pour l'identification des personnes lors d'enquêtes criminelles. Les traces papillaires peuvent être classées en trois catégories : les traces visibles, les traces moulées et les traces latentes. Lors de l'analyse d'une scène de crime, les enquêteurs cherchent à obtenir des copies portables et pérennes des traces papillaires, en procédant par exemple à une photographie de celles-ci. Les traces visibles peuvent être photographiées directement et les traces moulées peuvent être également photographiées sous certaines conditions d'éclairage.

Les traces latentes sont les plus nombreuses et les plus difficiles à traiter. En effet, elles sont peu visibles, voire invisibles à la lumière directe et il faut donc les faire apparaître au moyen de divers procédés, en augmentant le contraste entre elles et le support. Elles sont produites par le dépôt d'un mélange complexe d'humeurs naturelles sécrétées par trois types de glandes : les glandes sudoripares eccrines et apocrines et les glandes sébacées. Les glandes sudoripares produisent la sueur et sont réparties sur l'ensemble du corps. Les surfaces palmaires et plantaires de la peau sont exclusivement liées à des glandes eccrines dont les sécrétions sont évacuées par des pores situés sur le sommet des crêtes papillaires. Ces sécrétions sont composées de matières inorganiques telles que : eau (98%), chlorures, sulfates, phosphates, ammoniaque et ions métalliques. Elles comprennent aussi des matières organiques telles que : acides aminés, acide urique, acide lactique, urée, sucres, créatinine et choline. Les traces latentes sont donc à l'origine produites par le dépôt de sueur provenant de glandes eccrines. Elles sont souvent contaminées par les graisses provenant des glandes sébacées : le fait de se toucher le visage entraîne un mélange de sueur et de graisses à la surface des mains.

Les techniques de révélation ciblent donc les constituants produits par les glandes eccrines (notamment l'eau, les acides aminés et les chlorures), mais également les matières grasses présentes par contamination.

L'utilisation des cyanoacrylates pour la révélation des traces papillaires est un procédé universellement utilisé par les forces de l'ordre. C'est une technique simple d'exécution et particulièrement efficace. Cette méthode a par exemple été décrite par S. Moritomo et al ; ("A new method to enhance visualization of latent fingermarks by sublimating dyes..."; Forensic Science International; vol. 97, 1998, p101-108). Les cyanoacrylates sont une famille de molécules utilisées habituellement comme colles et souvent connus sous le nom de Superglue® ou Cyanolite®. Dans ce procédé, les cyanoacrylates entrent dans une réaction de polymérisation nucléophile. Tout site ou espèce suffisamment riche en électrons peut jouer le rôle du nucléophile initiateur de la réaction de polymérisation. En particulier, l'eau ou encore les fonctions -COOH des acides gras peuvent jouer ce rôle. Cette réaction de polymérisation permet notamment au cyanoacrylate de révéler des traces latentes au premier rang desquelles les empreintes papillaires. Cette réaction se fait par sublimation ou fumigation du cyanoacrylate. Les molécules de vapeurs provenant du cyanoacrylate se combinent aux dépôts sudoraux pour former une chaîne chimique (réaction de polymérisation) qui permet de colorer les crêtes des traces en blanc tout en les fixant. Cette technique est employée efficacement pour fixer les traces présentes sur la plupart des supports non poreux ou semi poreux, et plus particulièrement les plastiques souples ou rigides, les métaux, le verre, les bois vernis, peints ou glacés...

La principale limite de la technique de fumigation de cyanoacrylate est liée à la couleur blanche de la révélation. Le contraste entre la trace révélée et le support sur lequel est présente cette trace n'est pas toujours suffisant. Pour renforcer le contraste, la solution d'usage consiste à utiliser un éclairage rasant, à appliquer une poudre dactyloscopique ou encore un colorant après révélation. Une combinaison de plusieurs techniques est donc nécessaire pour obtenir un résultat satisfaisant. L'emploi de colorants, sous forme de poudres ou de solutions, présente l'avantage de s'adapter aux configurations rencontrées : la nature du colorant, sa couleur peuvent être choisie dans une gamme assez large afin de permettre une coloration optimale de la trace et un contraste performant.

Le document WO2008/020951 concerne un mélange ainsi qu'un procédé de révélation d'empreintes digitales latentes le mettant en oeuvre. Ledit mélange comprend un cyanoacrylate sous forme liquide, un réactif fluorescent soluble dans le cyanoacrylate (notamment le 2-(2-hydroxyphenyl)benzothiazole) et un solvant (notamment l'éthyl lactate), ledit mélange étant sublimable à une certaine température et capable de réagir avec un des composés constitutifs des empreintes digitales latentes pour obtenir une image fluorescente desdites empreintes.

Le document US 4,504,408 concerne un activateur de phase vapeur imprégné d'un colorant fluorescent et d'une composition contenant des solvants organiques chlorés. Ainsi, au contact d'un cyanoacrylate, l'ensemble se vaporise et génère une fumée fluorescente qui permet de révéler les empreintes digitales lorsqu'elles sont exposées à une lumière ultra-violette.

Malheureusement, nombre de ces procédés dégradent l'ADN, interdisant leur emploi avant la recherche de matériel biologique, et n'offrent pas toujours une solution satisfaisante (limite technique, temps, coût...).

Ainsi, les solutions habituellement préconisées ne sont pas toutes compatibles avec des examens ultérieurs que pourraient subir la trace ou le support sur lequel elle est déposée, et aucune ne permet de résoudre efficacement le problème de la résolution de traces pour l'ensemble des cas pratiques rencontrés, notamment quel que soit le support sur lequel est déposée la trace ou quels que soient les examens ultérieurs.

Les présents inventeurs, ont trouvé de façon inattendue et surprenante que les différents problèmes liés aux techniques antérieures pouvaient être résolus grâce à l'utilisation d'une trousse de révélation comprenant un agent fluorescent de la famille des tétrazines (décrits par exemple par GONG Y-A et al : "Synthesis and Physical Chemistry of tetrazines : which ones are fluorescent and why ?"; European Journal of Organic Chemistry vol. 2009, n°35, p6121-6128 et CLAVIER G et al: "s-tetrazines as building block for new functional molecules and molecular materials"; Chemical Review; vol. 110, n°6, p3299-3314) et un cyanoacrylate.

Ainsi la présente invention porte sur une trousse de révélation comprenant un agent fluorescent et un cyanoacrylate, un procédé de co-fumigation d'un agent fluorescent et d'un cyanoacrylate et l'utilisation de ce procédé pour la révélation de traces, notamment de traces papillaires.

Selon un premier objet, l'invention a pour objet une trousse de révélation comprenant :
(A) un cyanoacrylate; et
(B) un agent fluorescent choisi parmi les tétrazines de formule 1 : 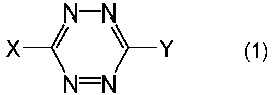 dans laquelle X et Y représentent indépendamment
   - un groupe électroattracteur non-ionique, pouvant être substitué par un groupe polymérisable comprenant une double liaison, une triple liaison ou un hétérocycle,
   - un groupe aziridine, éventuellement substitué par alkyle ou aryle;
   X et Y ne pouvant pas représenter un groupe amino ou tout autre groupement lié au cycle tétrazine par un atome d'azote, autre que ledit groupe aziridine; et
   X et Y ne pouvant pas représenter un groupe thio ou tout autre groupement lié au cycle tétrazine par un atome de soufre.

L'agent fluorescent présente un poids moléculaire inférieur à 400 g.mol⁻¹, de préférence de 100 à 300 g.mol⁻¹, plus préférentiellement de 130 à 250 g.mol⁻¹; une température de sublimation inférieure à 200°C, de préférence de 50 à 150°C, plus préférentiellement de 60°C à 120°C; et un coefficient d'extinction inférieur à 2950 L.mol⁻¹.cm⁻¹, de préférence de 200 à 2950 L.mol⁻¹.cm⁻¹, plus préférentiellement de 1000 à 2950 L.mol⁻¹.cm⁻¹.

Par « trousse de révélation » on entend un article comprenant les éléments (A) et (B), ceux-ci pouvant être sous forme séparée, sous forme d'un prémélange à diluer ou d'un mélange prêt à l'emploi.

Dans un mode de réalisation particulier, l'invention porte également sur une composition comprenant (A) un cyanoacrylate et (B) un agent fluorescent choisi parmi les tétrazines de formule 1 tels que définis précédemment. La composition selon l'invention peut comprendre un solvant.

La température de sublimation est déterminée par analyse thermogravimétrique (ATG). La rampe de température utilisée est de 1 °C/min.

Le coefficient d'extinction molaire est défini comme le rapport entre l'absorptivité et la concentration d'une entité chimique absorbante dans un milieu donné, par exemple le dichlorométhane, (exprimé en L·mol⁻¹·cm⁻¹) et est déterminé par spectroscopie d'absorption UV-Visible, de 250 à 800 nm, (mesure de l'absorbance de solutions de concentrations connues dans des cuves de trajet optique connus).

Avantageusement, l'agent fluorescent présente une brillance supérieure à 200 L.mol⁻¹.cm⁻¹, de préférence de 250 à 4000 L.mol⁻¹.cm⁻¹. La brillance est définie comme le produit du coefficient d'extinction molaire à la longueur d'excitation choisie par le rendement quantique de fluorescence; le coefficient d'extinction molaire étant déterminé par spectroscopie d'absorption UV-Visible, de 250 à 800 nm, et le rendement quantique de fluorescence étant déterminé par spectroscopie d'émission de fluorescence, selon les protocoles usuels connus de l'homme de l'art. Le rendement quantique de fluorescence du composé fluorescent étudié est déterminé en comparant l'intensité de fluorescence d'une solution de ce composé à l'intensité de fluorescence d'une solution isoabsorbante d'un fluorophore de référence (de rendement quantique connu). L'intensité de fluorescence du composé fluorescent étudié est déterminée à partir d'une solution de ce composé dans le dichlorométhane et la longueur d'onde d'excitation est choisie de façon à ce que, à cette longueur d'onde donnée, l'absorption dudit composé à étudier est supérieure ou égale à 10% de son maximum d'absorption sur un spectre d'absorption UV-visible. L'intensité de fluorescence du fluorophore de référence est déterminée à la même longueur d'onde donnée à partir d'une solution du fluorophore de référence dans le solvant pour lequel il est référencé. Les solutions du composé à étudier et du fluorophore de référence doivent être isoabsorbantes, c'est-à-dire que les concentrations sont ajustées de façon à ce que les deux solutions présentent une absorption équivalente à la longueur d'onde d'excitation donnée.

L'agent fluorescent est un agent fluorescent organique. Par agent fluorescent organique au sens de la présente invention, on entend toute molécule organique présentant en tant que telle des propriétés fluorescentes. Cela exclut par exemple les matériaux inorganiques et les chélates inorganiques ou organiques, tels que les chélates de lanthanide, de thorium ou d'yttrium.

Par le terme « groupe électroattracteur non ionique» au sens de la présente invention, on entend tout groupement ayant un effet inducteur attracteur d'électrons à l'exception des groupes ioniques, en particulier les halogènes et les groupes neutres constitués à partir d'un hétéroatome bi- ou trivalent comme l'oxygène, notamment -O-A-R où A représente une liaison simple ou un alkyldiyle en C₁-C₄ et R est alkyle, alcényle, alcynyle, cycloalkyle, aryle ou époxyalkyle.

Par le terme « halogène » au sens de la présente invention, on entend les atomes F, Cl, Br ou I, en particulier, F et Cl.

Par le terme « alkyle » au sens de la présente invention, on entend tout groupement alkyle linéaire ou branché, en particulier, les groupements alkyle contenant 1 à 6 atomes de carbone, notamment les groupes méthyle, éthyle, n-propyle, isopropyle ou isobutyle.

Par le terme « alcényle » au sens de la présente invention, on entend tout groupement alcényle comprenant au moins une double liaison, en particulier, les groupements alcényle contenant 2 à 6 atomes de carbone, notamment les groupes éthényle ou allyle.

Par le terme « alcynyle » au sens de la présente invention, on entend tout groupement alcynyle comprenant au moins une triple liaison, en particulier, les groupements alcynyle contenant 2 à 6 atomes de carbone, notamment les groupes éthynyle et propargyle.

Par le terme « cycloalkyle » au sens de la présente invention, on entend tout groupement cycloalkyle, en particulier les groupements cycloalkyle monocycliques, bicycliques ou tricycliques, chaque cycle contenant 5 ou 6 atomes de carbone notamment les groupes cyclohexyle ou adamantanyle.

Par les termes « aryle » (Ar) au sens de la présente invention on entend tout groupement aryle, en particulier les groupements aryle monocycliques, bicycliques ou tricycliques contenant 5 ou 6 atomes de carbone, notamment les groupes benzyle ou naphtalényle.

Par le terme « époxyalkyle » au sens de la présente invention, on entend tout groupement époxyalkyle, en particulier les groupements époxyalkyle contenant 2 à 6 atomes de carbone, notamment les groupes 2,3-époxypropyle ou 3,4-époxybutyle.

Par le terme « alkyldiyle en C₁-C₄ » au sens de la présente invention, on entend tout groupement alkyle bivalent contenant 1 à 4 atomes de carbone, notamment les groupes -CH₂- et -(CH₂)₂-.

Par groupe "aziridine", on entend le groupe cyclique à trois chainons -NC₂H₄. Les carbones de ce groupes pouvant être substitués par un groupe alkyle ou aryle.

Par "groupe polymérisable" au sens de la présente invention, on entend un groupe possédant une double liaison, une triple liaison ou un hétérocycle.

Selon un mode de réalisation particulier, X et Y sont choisis indépendamment dans le groupe constitué de halogène, azoture, cyano et -O-A-R où A représente une liaison simple ou un alkyldiyle en C₁-C₄, de préférence -CH₂-, et R est alkyle, alcényle, alcynyle, cycloalkyle, aryle ou époxyalkyle.

Selon un autre mode de réalisation particulier, X est choisi dans le groupe constitué de halogène, azoture, cyano et -O-A-R' où A représente une liaison simple ou un alkyldiyle en C₁-C₄,de préférence -CH₂-, et R' est alkyle, cycloalkyle ou aryle, et Y est -OR" où R" est alcényle, alcynyle ou époxyalkyle.

De manière avantageuse, chacun des substituants X et Y comprend au plus 12 atomes de carbone.

Selon un mode de réalisation particulier, la tétrazine de formule 1 est choisie dans le groupe constitué par la 3,6-dichloro-1,2,4,5-tétrazine, la 3-chloro-6-méthoxy-1,2,4,5-tétrazine, la 3-chloro-6-éthoxy-1,2,4,5-tétrazine, la 3,6-diméthoxy-1,2,4,5-tétrazine, la 3-chloro-6-(prop-2-ényloxy)-1,2,4,5-tétrazine, la 3-chloro-6-(prop-2ynyloxy)-1,2,4,5-tétrazine, la 3-chloro-6-(adamant-2-yloxy)-1,2,4,5-tétrazine, la 3-chloro-6-(adamant-1-ylméthoxy)-1,2,4,5-tétrazine, la 3-chloro-6-(naphtalèn-1-yloxy)-1,2,4,5-tétrazine, la 3-chloro-6-(3-époxybutyloxy)-1,2,4,5-tétrazine, le produit de formule 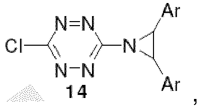 et leurs mélanges.
Le tableau 1 présente les caractéristiques de certains de ces composés :

**Tableau 1**

| Composé | Poids moléculaire (g.mol⁻¹) | Température de sublimation (°C) | Coefficient d'extinction* (L.mol⁻¹.cm⁻¹) | Brillance* (L.mol⁻¹.cm⁻¹) |
|---|---|---|---|---|
| 3,6-dichloro-1,2,4,5-tétrazine | 150,95 | 76 | 1900 | 270 |
| 3-chloro-6-méthoxy-1,2,4,5-tétrazine | 146,54 | 86 | 2905 | 1100 |
| 3-chloro-6-éthoxy-1,2,4,5-tétrazine | 160,56 | 87 | 2900 | 780 |
| 3,6-diméthoxy-1,2,4,5-tétrazine | 142,12 | 99 | 2935 | 320 |
| 3-chloro-6-(adamant-1-ylméthoxy)-1,2,4,5-tétrazine | 280,75 | 138 | 2950 | 1180 |

| | | | | |
|---|---|---|---|---|
| mesuré dans le dichlorométhane | | | | |

Le cyanoacrylate utilisé dans la trousse de révélation selon l'invention peut être tout cyanoacrylate communément connu et disponible commercialement pour une application en tant qu'adhésif instantané. En particulier, les cyanoacrylates pouvant être utilisés dans la trousse de révélation selon l'invention sont les cyanoacrylates de formule 2 : 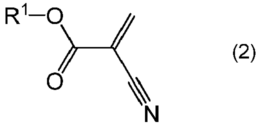 dans laquelle R¹ est alkyle, alcényle, alcynyle, cycloalkyle ou aryle, R¹ pouvant être éventuellement substitué par un halogène ou un groupe hydroxy. Par exemple, le cyanoacrylate est choisi dans le groupe constitué par le 2-cyanoacrylate de méthyle, le 2-cyanoacrylate d'éthyle, le 2-cyanoacrylate de propyle, le 2-cyanoacrylate de butyle, le 2-cyanoacrylate d'allyle, le 2-cyanoacrylate de méthoxyéthyle, le 2-cyanoacrylate d'éthoxyéthyle, le 2-cyanoacrylate de 2-chloroéthyle, le 2-cyanoacrylate de cyclohexyle, le 2-cyanoacrylate d'éthoxycarbonylméthyle, le 2-cyanoacrylate de trifluoroéthyle et leurs mélanges.

Les cyanoacrylates particulièrement préférés sont les cyanoacrylates présentant une faible viscosité. Selon un mode de réalisation particulier, le cyanoacrylate est choisi parmi les cyanoacrylates d'alkyle inférieur (alkyle en C1-C4), notamment le 2-cyanoacrylate d'éthyle ou le 2-cyanoacrylate de méthyle.

Les composés (A) et (B) peuvent être présents dans la trousse de révélation séparément ou sous la forme d'un mélange.

De façon avantageuse, lorsque l'agent fluorescent est la 3-chloro-6-méthoxy-1,2,4,5-tétrazine ou la 3-chloro-6-(prop-2-ynyloxy)-1,2,4,5-tétrazine, il est présent sous une forme séparé du cyanoacrylate, c'est-à-dire que les deux produits ne sont pas en contact l'un avec l'autre.

Selon un autre mode de réalisation, l'agent fluorescent et le cyanoacrylate sont en mélange, la trousse de révélation est alors une composition de révélation comprenant chacun des composés (A) et (B).

Ce mode de réalisation, est particulièrement adapté lorsque l'agent fluorescent est la 3-chloro-6-éthoxy-1,2,4,5-tétrazine. Selon un mode de réalisation avantageux, la composition de révélation comprend la 3-chloro-6-éthoxy-1,2,4,5-tétrazine et l'éthyl-2-cyanoacrylate.

Dans la trousse de révélation ou la composition de révélation, le ratio en poids des composés (A) et (B) est de 100 :1 à 500 :1. Dans une première alternative, le ratio (A):(B) est de préférence de 200 :1 à 400 :1. Dans une deuxième alternative, le ratio (A):(B) est de préférence de 100 :1 à 200 :1.

Selon un autre aspect, l'invention porte sur un procédé de co-fumigation. d'un cyanoacrylate (A) et d'un agent fluorescent (B) tels que définis ci-dessus. Ce procédé permet de déposer une couche polymère fluorescente sur une surface.

Ce procédé est unique du fait de la facilité de sublimation de l'agent fluorescent (B). En effet, du fait de leur température de sublimation relativement élevée, voire très élevée, les colorants organiques habituels sont dégradés lors de leur fumigation. Au contraire, l'agent fluorescent (B) selon l'invention présente une température de sublimation relativement faible, caractéristique unique pour un colorant organique, qui est principalement due à leur faible poids moléculaire. Ainsi, il est possible d'effectuer la co-fumigation d'un ester de cyanoacrylate et de l'agent fluorescent selon l'invention sans que ce dernier soit dégradé.

Le cyanoacrylate (A) et l'agent fluorescent (B) mis en oeuvre dans le procédé selon l'invention sont tels que définis ci-dessus en lien avec la trousse de révélation.

Le procédé de co-fumigation selon l'invention comprend la fumigation simultanée du cyanoacrylate (A) et de l'agent fluorescent (B) et le dépôt sur une surface du polymère fluorescent ainsi formé.

Le procédé de co-fumigation est effectué dans des conditions telles qu'il permette la vaporisation ou la sublimation du cyanoacrylate (A) et de l'agent fluorescent (B) tout en évitant la dégradation de ces composés. La température de co-fumigation sera aisément déterminée par l'homme du métier en fonction des températures de vaporisation ou de sublimation du cyanoacrylate et de l'agent fluorescent utilisés. Des températures distinctes peuvent être mises en oeuvre si besoin. Cependant, on préférera mettre en oeuvre une tétrazine et un cyanoacrylate présentant des températures de sublimation ou de vaporisation compatibles, c'est-à-dire des températures de sublimation ou de vaporisation n'entrainant pas la décomposition de l'autre composé. Par exemple, la co-fumigation est effectuée entre 80 et 120°C. La durée de la co-fumigation est telle qu'elle permette le dépôt d'une couche polymère fluorescente sur la surface du support, en particulier sur les traces présentes sur le support. Typiquement, le temps de co-fumigation est supérieur à 5 min, de préférence il est compris entre 10 et 30 min. Le cyanoacrylate (A) et l'agent fluorescent (B) sont utilisés simultanément lors de la co-fumigation mais ils peuvent être présents sous forme de deux composés (A) et (B) séparés. Cependant, on préfèrera mettre en oeuvre la co-fumigation à partir d'un mélange des composés (A) et (B). En effet, les dispositifs de fumigation courants ne possèdent souvent pas de moyens de chauffage permettant une co-fumigation simultanée de deux composés séparés, qui plus est à des températures distinctes. De plus, Il est plus aisé de parvenir à une fumigation simultanée à partir d'un mélange qu'en réalisant une co-fumigation des composés pris séparément. Enfin, la co-fumigation des composés (A) et (B) à partir d'un mélange de ceux-ci permet d'obtenir un dépôt plus homogène. Il est néanmoins essentiel que, dans le cas d'une co-fumigation à partir d'un mélange des composés (A) et (B), ceux-ci aient une température de vaporisation ou de sublimation voisine, typiquement entre 80 et 120 °C. Le ratio en poids entre la quantité de cyanoacrylate (A) et la quantité d'agent fluorescent (B) est compris entre 100 :1 et 500 :1, de préférence entre 100 :1 et 200 :1.

Ce procédé de co-fumigation provoque une réaction de polymérisation nucléophile qui est initiée par tout site ou espèce suffisamment riche en électrons. En particulier, l'eau peut jouer ce rôle. Ainsi, selon un mode de réalisation avantageux, le procédé selon l'invention est conduit dans une atmosphère humide, notamment avec un taux d'humidité de l'air ambiant d'au moins 70%, de préférence 80%. La facilité de mise en oeuvre de ce procédé, ainsi que la tenue exceptionnelle du revêtement due aux propriétés d'adhérence bien connues des esters de cyanoacrylates en font un procédé de choix pour la réalisation de revêtements fluorescents résistants, en particulier pour des objets à géométrie complexe.

Du fait de la bonne qualité du dépôt réalisé et de sa fluorescence, le procédé de l'invention peut être mis en oeuvre sur n'importe quel type de support, y compris des supports poreux. Ce procédé est donc applicable quel que soit le type de support, tel que les plastiques souples ou rigides, les métaux, le verre, les bois vernis, peints ou glacés, l'épiderme (peau).

Le procédé selon l'invention peut permettre la révélation de traces déposées sur un support, notamment de traces papillaires latentes. L'invention a donc également pour objet l'utilisation du procédé de co-fumigation décrit ci-dessus pour la révélation de traces, notamment de traces papillaires latentes sur un support. Ce procédé peut s'appliquer à différents échantillons constitués de supports de différente nature couramment prélevés sur les scènes de crime et comportant des traces papillaires latentes, par exemple des empreintes digitales et palmaires présentes sur des échantillons en verre (bouteille, lame mince de laboratoire), en plastique (sac poubelle, sac de congélation, cellophane, compact disc) ou pourvus d'une surface métallique (canette), ou encore à la scène de crime elle-même lorsqu'il s'agit d'un espace clos, par exemple une pièce.

Dans un mode de réalisation particulier, l'invention a pour objet un procédé de révélation de traces sur un support comprenant les étapes consistant à :
- présenter ledit support portant des traces à un dispositif comprenant des moyens de fumigation et contenant un cyanoacrylate (A) et un agent fluorescent (B) tels que définis ci-dessus;
- co-fumiger les composés (A) et (B) selon le procédé tel que défini ci-dessus à l'aide dudit dispositif de façon à obtenir un support fumigé;
- soumettre ledit support fumigé à une lumière ultraviolette.

Avantageusement, le procédé de révélation de traces selon l'invention comprend les étapes consistant à :
- présenter ledit support portant des traces à un dispositif comprenant des moyens de fumigation et contenant un cyanoacrylate (A) et une tétrazine (B) de formule 1 telle que définie ci-dessus ;
- mettre en oeuvre le procédé de co-fumigation tel que décrit ci-dessus par ledit dispositif de façon à obtenir un support fumigé;
- soumettre ledit support fumigé à une lumière ultraviolette.

Ce procédé de révélation peut être mis en oeuvre par différents dispositifs comprenant des moyens de fumigation bien connus de l'homme du métier, notamment un dispositif de fumigation de laboratoire (tel qu'une cabine de fumigation du type UCV 1000 commercialisé par Foster&Freeman) pour l'analyse en laboratoire d'objets recueillis sur les scène de crime, un dispositif de fumigation portatif (du type CyanoWand®) pour une analyse localisée sur une scène de crime, ou un dispositif de fumigation d'une pièce de bâtiment entière (du type SUPERFume® commercialisé par Foster&Freeman) pour l'analyse complète d'une scène de crime en intérieur. Le support peut être donc selon le cas un objet, une partie d'un objet ou d'une surface, ou une pièce entière incluant toutes les cloisons, sol, plafond, menuiseries ou tout objet qui s'y trouverait.

Les traces peuvent être des traces papillaires latentes.

Le cyanoacrylate (A) et l'agent fluorescent (B) sont tels que définis précédemment en relation avec la trousse de révélation.

Avant de mettre en oeuvre le procédé de co-fumigation, en particulier lorsque celui-ci est mis en oeuvre dans une cabine de fumigation, l'atmosphère est humidifiée pour favoriser l'amorçage de la réaction de polymérisation nucléophile. De manière avantageuse, l'atmosphère est humidifiée de façon à atteindre un taux d'humidité d'au moins 70%, de préférence 80%.

La quantité de cyanoacrylate (A) et d'agent fluorescent (B) à utiliser pour le procédé de révélation de traces selon l'invention dépend de plusieurs paramètres tels que le volume à fumiger (par exemple le volume de la cabine de fumigation ou le la pièce à fumiger), la durée du cycle de fumigation, le volume et la surface des objets soumis à la fumigation, ou le degré et la qualité de la révélation attendu.

La détection des traces par rayonnement ultraviolet se fait à une longueur d'ondes comprise entre 250 et 370 nm, de préférence aux alentours de 365 nm ou 312nm, à l'aide d'un éclairage adapté, par exemple une lampe standard de labo 365 nm ou 312nm ou un tube néon 10W 365 nm ou 312 nm. Une lumière filtrée comprise entre 450 et 535 nm, de préférence aux alentours de 515 nm, permet également la détection desdites traces.

Dans un premier mode de réalisation, le procédé de révélation de traces sur un support selon l'invention, comprend les étapes consistant à :
- introduire ledit support portant des traces dans une cabine de fumigation contenant un cyanoacrylate (A) et l'agent fluorescent (B) tels que définis ci-dessus ;
- co-fumiger les composés (A) et (B) selon le procédé tel que décrit ci-dessus de façon à obtenir un support fumigé;
- soumettre ledit support fumigé à une lumière ultraviolette.

La cabine de fumigation est hermétique. Elle présente un dispositif permettant de régler l'humidité ambiante. Elle est dotée d'au moins un creuset dont la température peut être régulée. Selon un mode de réalisation particulier, elle est dotée de deux creusets présentant chacun indépendamment un dispositif de régulation de la température.

Elle peut également être dotée d'un dispositif de décontamination de traces biologiques afin d'éviter toute contamination du support à analyser et préserver celui-ci pour une éventuelle recherche d'ADN ultérieure.

La quantité de produit déposé par fumigation doit pouvoir également être contrôlée avec une certaine précision. A cet effet, un objet témoin est généralement introduit dans la cabine de fumigation avec le support à analyser pour servir d'indicateur pour le temps d'exposition aux fumées.

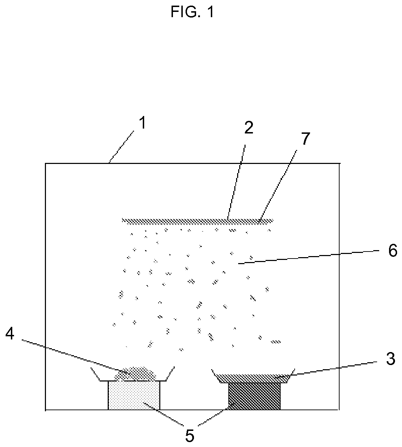

La figure 1 est une représentation schématique de la mise en oeuvre de l'étape de co-fumigation du procédé de révélation de traces selon le premier mode de réalisation du procédé de révélation de traces de l'invention (cas d'une cabine de fumigation à deux creusets). Dans ce mode de réalisation, le support 2 est introduit dans l'enceinte de la cabine de fumigation 1. Le cyanoacrylate 3 et l'agent fluorescent 4 sont disposés dans des creusets 5. Lors de l'étape de co-fumigation, le cyanoacrylate 3 et l'agent fluorescent 4 sont vaporisés ou sublimés simultanément sous l'effet de la température et forment un mélange de vapeurs 6. Au contact du support 2, et en particulier des traces présentes sur le support, ce mélange de vapeur 6 forme un revêtement fluorescent 7 composé d'un polymère cyanoacrylique dans lequel sont incorporées des molécules d'agent fluorescent.

Dans un deuxième mode de réalisation, le procédé de révélation de traces sur un support selon l'invention, comprend les étapes consistant à :
- présenter ledit support portant des traces à un dispositif de fumigation portatif individuel contenant un cyanoacrylate (A) et l'agent fluorescent (B) tels que définis ci-dessus ;
- co-fumiger les composés (A) et (B) à l'aide du dispositif de fumigation portatif selon le procédé tel que décrit ci-dessus de façon à obtenir un support fumigé;
- soumettre ledit support fumigé à une lumière ultraviolette.

Dans un troisième mode de réalisation, le procédé de révélation de traces dans une pièce selon l'invention comprend les étapes consistant à :
- disposer le dispositif de fumigation, contenant un cyanoacrylate (A) et l'agent fluorescent (B) tels que définis ci-dessus , dans une pièce fermée;
- co-fumiger les composés (A) et (B) à l'aide du dispositif de fumigation de façon à obtenir une pièce fumigée;
- soumettre ladite pièce fumigée à une lumière ultraviolette.

Par l'application du procédé de l'invention, l'agent fluorescent est directement incorporé dans le dépôt de cyanoacrylate. Les traces révélées sur le support peuvent donc être observées notamment sous rayonnement ultraviolet adapté aux caractéristiques de fluorescence de l'agent fluorescent utilisé, par exemple à une longueur d'ondes comprise entre 300 et 370 nm, en particulier 365 nm ou 312 nm, afin d'offrir un contraste optimal. Le procédé de l'invention permet donc de révéler efficacement toute trace papillaire latente en une seule étape. L'emploi de colorants ou de poudres dactyloscopiques post-révélation n'est donc plus nécessaire. Contrairement à l'emploi de colorants, fluorescents ou non, en post-révélation qui nécessite un post-traitement thermique pour réaliser le dépôt du colorant ou améliorer l'adhérence de celui-ci sur le dépôt d'acrylate, le procédé de l'invention permet de limiter les manipulations nécessaires à la révélation des traces et ainsi de garantir un maximum de compatibilité dans le cadre de la recherche d'ADN sur le support, en effet, l'agent fluorescent (B) choisi parmi les tétrazines de formule 1, ne réagissent pas avec l'ADN éventuellement présent sur le support.

Un des avantages essentiels du procédé de l'invention est par conséquent de préserver la molécule d'ADN. Ceci représente un atout majeur pour les enquêtes criminelles où les techniques d'investigation scientifiques sont de plus en plus exigeantes et où la complémentarité et la compatibilité des procédés est requise.

De plus, le procédé de co-fumigation selon l'invention permet d'obtenir une fluorescence sélective des traces à révéler. En effet, sans être lié à une quelconque théorie, il est supposé que l'agent fluorescent réagit avec le cyanoacrylate de sorte que l'agent fluorescent est lié au dépôt cyanoacrylate. La fluorescence des dépôts de cyanoacrylate est par conséquent persistante alors que la fluorescence des dépôts d'agent fluorescent libre autour des traces disparaît rapidement du fait de la volatilité élevée de l'agent fluorescent. Il en résulte une distinction facilitée desdites traces à révéler, fluorescentes, par rapport au support, non fluorescent. Au contraire, lorsque le dépôt est obtenu en réalisant une imprégnation du dépôt de cyanoacrylate (par exemple un post-traitement avec un colorant fluorescent ou une co-fumigation avec un agent fluorescent ne réagissant pas avec le cyanoacrylate), la fluorescence apparaît à la fois sur le dépôt de cyanoacrylate et sur le substrat et la différence de contraste entre les traces à révéler et le substrat est beaucoup moins important.

Le dépôt obtenu est également particulièrement stable du point de vue de la fluorescence. En effet, l'agent fluorescent étant lié au dépôt cyanoacrylate, celui-ci ne se sublime pas et la fluorescence est particulièrement stable dans le temps. Typiquement, le dépôt de cyanoacrylate obtenu par le procédé de l'invention reste fluorescent pendant au moins 6 mois. La fluorescence du dépôt est d'autant plus durable dans le temps que les conditions de conservation permettent de prévenir la dégradation de l'agent fluorescent. Ainsi, la fluorescence du dépôt est particulièrement stable lorsqu'il est conservé à l'abri de la lumière et de l'humidité.

L'invention porte également sur l'utilisation de la trousse de révélation selon l'invention pour la mise en oeuvre du procédé de co-fumigation et du procédé de révélation de traces décrits ci-dessus. L'invention porte également sur l'utilisation de la trousse de révélation selon l'invention pour la révélation de traces sur un support.

Les exemples suivants illustrent davantage le procédé de l'invention et son intérêt. Ces exemples ne sont présentés que dans un but d'illustration et ne peuvent être considérés comme limitatifs de l'invention.

### Exemples:

L'ester de cyanoacrylate utilisé est un cyanoacrylate commercialisé par Foster&Freeman sous la marque Cyanobloom® comprenant >99% de 2-cyanoacrylate d'éthyle et <0,02% de 1,4-dihydroxybenzène quinol.

### Exemple 1:

Un mélange de 10mg de 3-chloro-6-éthoxy-1,2,4,5-tétrazine dans 0,375g de cyanoacrylate a été préparé et disposé dans le creuset d'une cabine de fumigation modèle MCV 1000 Foster&Freeman. L'échantillon à analyser, qui consistait en un verre à boire portant des empreintes digitales, a été introduit dans la cabine de fumigation. Le degré d'hygrométrie dans la cabine de fumigation a été monté à 80% et le creuset a été chauffé progressivement jusqu'à une température de 120°C.

Après 15 min de fumigation, l'échantillon a été sorti de la cabine de fumigation et il a été observé sous un rayonnement ultraviolet à 312nm.

Après révélation, des traces particulièrement bien distinctes de couleur jaune-orangé ont été observées au rayonnement ultraviolet à 312nm pour chacun des échantillons analysés.

Ces échantillons ont été conservés à l'abri de la lumière et le l'humidité. 6 mois après leur révélation, les traces pouvaient être observées de façon tout aussi distincte sous un rayonnement UV à 312nm.

### Exemple 2:

L'exemple 1 a été reproduit en utilisant 10mg de 3-chloro-6-méthoxy-1,2,4,5-tétrazine à la place des 10mg de 3-chloro-6-éthoxy-1,2,4,5-tétrazine, mais en disposant la tétrazine et le cyanoacrylate dans deux creusets séparés dans la cabine de fumigation.

La température du creuset contenant le cyanoacrylate a été chauffé progressivement jusqu'à 120°C. Lorsque sa température a atteint environ 85-95°C, le creuset contenant la tétrazine a été chauffé progressivement jusqu'à 90°C. Ainsi, les deux composés ont pu fumiger simultanément.

Les échantillons analysés ont été observés sous un rayonnement ultraviolet à 312nm. Des traces particulièrement bien distinctes de couleur jaune-orangé ont été observées pour chaque échantillon.

Les traces pouvaient être observées de façon tout aussi distincte sous un rayonnement UV à 312nm même 6 mois après leur révélation et conservation à l'abri de la lumière et le l'humidité.

### Exemple 3:

L'exemple 2 a été reproduit en utilisant 10mg de 3-chloro-6-(prop-2-ynyloxy)-1,2,4,5-tétrazine à la place des 10mg de 3-chloro-6-méthoxy-1,2,4,5-tétrazine.

Les échantillons analysés ont été observés sous un rayonnement ultraviolet à 312nm. Des traces particulièrement bien distinctes de couleur jaune-orangé ont été observées pour chaque échantillon.

Ces échantillons ont été conservés à l'abri de la lumière et le l'humidité. 6 mois après leur révélation, les traces pouvaient être observées de façon tout aussi distincte sous un rayonnement UV à 312nm.

### Exemple 4

Les exemples 1 à 3 ont été reproduits avec un mélange de 10 mg de 3-chloro-6-éthoxy-1,2,4,5-tétrazine, 3-chloro-6-méthoxy-1,2,4,5-tétrazine ou 3-chloro-6-(prop-2-ynyloxy)-1,2,4,5-tétrazine respectivement dans 1 g de cyanoacrylate.

Les échantillons analysés ont été observés sous un rayonnement ultraviolet à 312nm. De la même manière, des traces particulièrement bien distinctes de couleur jaune-orangé ont été observées pour chaque échantillon. Ces traces pouvaient être observées de façon tout aussi distincte sous un rayonnement UV à 312nm même 6 mois après leur révélation, les échantillons ayant été conservés à l'abri de la lumière et de l'humidité.

## Revendications

1. Trousse de révélation comprenant :
(A) un cyanoacrylate ; et
(B) un agent fluorescent choisi parmi les tétrazines de formule 1 : 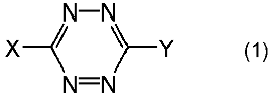 dans laquelle X et Y représentent indépendamment
- un groupe électroattracteur non-ionique, pouvant être substitué par un groupe polymérisable comprenant une double liaison, une triple liaison ou un hétérocycle,
- un groupe aziridine, éventuellement substitué par alkyle ou aryle;
X et Y ne pouvant pas représenter un groupe amino ou tout autre groupement lié au cycle tétrazine par un atome d'azote, autre que ledit groupe aziridine; et
X et Y ne pouvant pas représenter un groupe thio ou tout autre groupement lié au cycle tétrazine par un atome de soufre.

2. Trousse selon la revendication 1, **caractérisée en ce que** X et Y sont choisis indépendamment dans le groupe constitué des halogènes, azoture (azide), cyano et -OR où R
est alkyle, alcényle, alcynyle, cycloalkyle, aryle ou époxyalkyle.

3. Trousse selon l'une quelconque des revendications 1 ou 2, **caractérisée en ce que** la tétrazine de formule 1 est choisie dans le groupe constitué par la 3,6-dichloro-1,2,4,5-tétrazine, la 3-chloro-6-méthoxy-1,2,4,5-tétrazine, la 3-chloro-6-éthoxy-1,2,4,5-tétrazine, la 3,6-diméthoxy-1,2,4,5-tétrazine, la 3-chloro-6-(prop-2-ényloxy)-1,2,4,5-tétrazine, la 3-chloro-6-(prop-2-ynyloxy)-1,2,4,5-tétrazine, la 3-chloro-6-(adamant-2-yloxy)-1,2,4,5-tétrazine, la 3-chloro-6-(adamant-1-ylméthoxy)-1,2,4,5-tétrazine, la 3-chloro-6-(naphtalèn-1-yloxy)-1,2,4,5-tétrazine et la 3-chloro-6-(3-époxybutyloxy)-1,2,4,5-tétrazine, le produit de formule 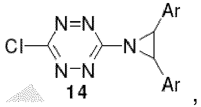 et leurs mélanges.

4. Trousse selon l'une quelconque des revendications 1 à 3, **caractérisée en ce que** le cyanoacrylate (A) est un cyanoacrylate de formule 2 : 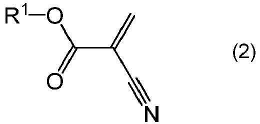 dans laquelle R¹ est alkyle, alcényle, alcynyle, cycloalkyle ou aryle, R¹ pouvant être éventuellement substitué par un halogène ou un groupe hydroxy.

5. Trousse selon l'une quelconque des revendications 1 à 4, **caractérisée en ce que** le cyanoacrylate (A) est choisi dans le groupe constitué par le 2-cyanoacrylate de méthyle, le 2-cyanoacrylate d'éthyle, le 2-cyanoacrylate de propyle, le 2-cyanoacrylate de butyle, le 2-cyanoacrylate d'allyle, le 2-cyanoacrylate de méthoxyéthyle, le 2-cyanoacrylate d'éthoxyéthyle, le 2-cyanoacrylate de 2-chloroéthyle, le 2-cyanoacrylate de cyclohexyle, le 2-cyanoacrylate d'éthoxycarbonylméthyle, le 2-cyanoacrylate de trifluoroéthyle et leurs mélanges.

6. Composition comprenant (A) un cyanoacrylate et (B) un agent fluorescent tels que définis dans les revendications 1 à 5.

7. Procédé de co-fumigation comprenant la co-fumigation simultanée d'un cyanoacrylate (A) et d'un agent fluorescent (B) tels que définis dans les revendications 1 à 5 et le dépôt sur une surface du polymère fluorescent ainsi formé.

8. Procédé selon la revendication 7, **caractérisé en ce que** le ratio en poids entre la quantité de cyanoacrylate (A) et la quantité d'agent fluorescent (B) est de 100 :1 à 500 :1, de préférence de 100 :1 à 200 :1.

9. Procédé selon l'une quelconque des revendications 7 ou 8, **caractérisé en ce que** la température de co-fumigation est comprise entre 80° et 120 °C.

10. Procédé de révélation de traces sur un support comprenant les étapes consistant à :
- présenter ledit support portant des traces à un dispositif comprenant des moyens de fumigation et contenant un cyanoacrylate (A) et un agent fluorescent (B) tels que définis dans les revendications 1 à 5;
- co-fumiger les composés (A) et (B) selon le procédé tel que défini dans les revendications 7 à 9 à l'aide dudit dispositif de façon à obtenir un support fumigé;
- soumettre ledit support fumigé à une lumière ultraviolette.

11. Utilisation de la trousse de révélation selon l'une quelconque des revendications 1 à 5 ou d'une composition selon la revendication 6 pour la révélation de traces sur un support.

## Patentansprüche

1. Enthüllungskit, umfassend:
(A) ein Cyanacrylat; und
(B) einen fluoreszierenden Wirkstoff, der ausgewählt ist unter den Tetrazinen mit folgender Formel 1: 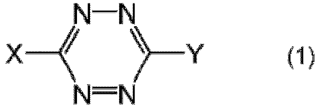 wobei X und Y unabhängig darstellen:
- eine nicht ionische elektronenziehende Gruppe, die durch eine polymerisierbare Gruppe, umfassend eine Doppelbindung, eine Dreifachbindung oder einen Heterozyklus, substituiert werden kann,
- eine Aziridin-Gruppe, eventuell substituiert durch Alkyl oder Aryl;
wobei X und Y nicht eine Aminogruppe oder jede andere Gruppierung darstellen können, die mit dem Tetrazin-Zyklus durch ein anderes Stickstoffatom als die Aziridin-Gruppe verbunden ist; und
wobei X und Y nicht eine Thiogruppe oder jede andere Gruppierung darstellen können, die mit dem Tetrazin-Zyklus durch ein Schwefelatom verbunden ist.

2. Kit nach Anspruch 1, **dadurch gekennzeichnet, dass** X und Y unabhängig in der Gruppe ausgewählt sind, die von den Halogenen, Azid, Cyanid und -OR gebildet ist, wobei R Alkyl, Alkenyl, Alkinyl, Cycloalkyl, Aryl oder Epoxyalkyl ist.

3. Kit nach einem der Anspruche 1 oder 2, **dadurch gekennzeichnet, dass** das Tetrazin der Formel 1 in der Gruppe ausgewählt ist, die von 3,6-Dichlor-1,2,4,5-Tetrazin, 3-Chlor-6-Methoxy-1,2,4,5-Tetrazin, 3-Chlor-6-Ethoxy-1,2,4,5-Tetrazin, 3,6-Dimethoxy-1,2,4,5-Tetrazin, 3-Chlor-6-(Prop-2-enyloxy)-1,2,4,5-Tetrazin, 3-Chlor,6-(Prop-2-inyloxy)-1-2-4-5-Tetrazin, 3-Chlor-6-(Adamant-2-yloxy)-1,2,4,5-Tetrazin, 3-Chlor-6-(Adamant-1-ylmethoxy)-1,2,4,5-Tetrazin, 3-Chlor-6-(Naphthalen-1-yloxy)-1,2,4,5-Tetrazin und 3-Chlor-6-(-3-Epoxybutyloxy)-1,2,4,5-Tetrazin, dem Produkt der Formel 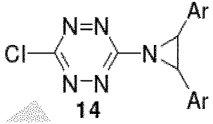 und ihren Gemischen gebildet ist.

4. Kit nach einem der Ansprüche 1 bis 3, **dadurch gekennzeichnet, dass** das Cyanacrylat (A) ein Cyanacrylat mit folgender Formel 2 ist: 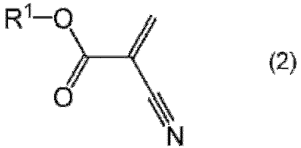 wobei R¹ Alkyl, Alcenyl, Alcynyl, Cycloalkyl oder Aryl ist, wobei R¹ eventuell durch ein Halogen oder eine Hydroxy-Gruppe substituiert sein kann.

5. Kit nach einem der Ansprüche 1 bis 4, **dadurch gekennzeichnet, dass** das Cyanacrylat (A) in der Gruppe ausgewählt ist, die von 2-Methyl-Cyanacrylat, 2-Ethyl-Cyanacrylat, 2-Propyl-Cyanacrylat, 2-Butyl-Cyanacrylat, 2-Allyl-Cyanacrylat, 2-Methoxyethyl-Cyanacrylat, 2-Ethoxyethyl-Cyanacrylat, 2-Cyanacrylat von 2-Chlorethyl, 2-Cyclohexyl-Cyanacrylat, 2-Ethoxycarbonylmethyl-Cyanacrylat, 2-Trifluorethyl-Cyanacrylat und ihren Gemischen gebildet ist.

6. Zusammensetzung, umfassend (A) ein Cyanacrylat und (B) einen fluoreszierenden Wirkstoff, wie in den Ansprüchen 1 bis 5 definiert.

7. Co-Begasungsverfahren, umfassend die gleichzeitige Co-Begasung eines Cyanacrylats (A) und eines fluoreszierenden Wirkstoffes, wie in den Ansprüchen 1 bis 5 definiert, und die das Aufbringen auf einer Oberfläche des so gebildeten fluoreszierenden Polymers.

8. Verfahren nach Anspruch 7, **dadurch gekennzeichnet, dass** das Gewichtsverhältnis zwischen der Menge an Cyanacrylat (A) und der Menge an fluoreszierendem Wirkstoff (B) 100:1 bis 500:1, vorzugsweise 100:1 bis 200:1, beträgt.

9. Verfahren nach einem der Ansprüche 7 oder 8, **dadurch gekennzeichnet, dass** die Temperatur der Co-Begasung zwischen 80° und 120°C liegt.

10. Verfahren zur Enthüllung von Spuren auf einem Träger, umfassend die folgenden Schritte, darin bestehend:
- den Träger, der die Spuren trägt, in eine Vorrichtung einzubringen, die Begasungsmittel umfasst und ein Cyanacrylat (A) und einen fluoreszierenden Wirkstoff (B), wie in den Ansprüchen 1 bi s5 definiert, enthält;
- die Verbindungen (A) und (B) nach dem Verfahren, wie in den Ansprüchen 7 bis 9 definiert, mit Hilfe der Vorrichtung zu cobegasen, um einen begasten Träger zu erhalten;
- den begasten Träger dem UV-Licht auszusetzen.

11. Verwendung des Enthüllungskits nach einem der Ansprüche 1 bis 5 oder einer Zusammensetzung nach Anspruch 6 zur Enthüllung von Spuren auf einem Träger.

## Claims

1. Visualization kit comprising:
(A) a cyanoacrylate; and
(B) a fluorescent agent selected from tetrazine of formula 1: 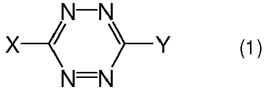 in which X and Y represent independently
- a nonionic, electron-withdrawing group, which may be substituted by a polymerizable group;
- an aziridine group, which is optionally substituted by alkyl or aryl;
X and Y cannot represent an amino group or any other group bonded to the tetrazine ring by a nitrogen atom, other than the said aziridine group; and
X and Y cannot represent a thio group or any other group bonded to the tetrazine ring by a sulphur atom.

2. Kit according to claim 1, **characterized in that** X and Y are selected independently from the group consisting of halogens, azide, cyano and -OR in which R is alkyl, alkenyl, alkynyl, cycloalkyl, aryl or epoxyalkyl.

3. Kit according to either of claims 1 or 2, **characterized in that** the tetrazine of formula 1 is selected from the group consisting of 3,6-dichloro-1,2,4,5-tetrazine, 3-chloro-6-methoxy-1,2,4,5-tetrazine, 3-chloro-6-ethoxy-1,2,4,5-tetrazine, 3,6-dimethoxy-1,2,4,5-tetrazine, 3-chloro-6-(prop-2-enyloxy)-1,2,4,5-tetrazine, 3-chloro-6-(prop-2-ynyloxy)-1,2,4,5-tetrazine, 3-chloro-6-(adamant-2-yloxy)-1,2,4,5-tetrazine, 3-chloro-6-(adamant-1-ylmethoxy)-1,2,4,5-tetrazine, 3-chloro-6-(naphthalen-1-yloxy)-1,2,4,5-tetrazine and 3 chloro-6-(3-epoxybutyloxy)-1,2,4,5-tetrazine, the product of formula 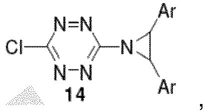 and mixtures thereof.

4. Kit according to any of claims 1 to 3, **characterized in that** the cyanoacrylate (A) is a cyanoacrylate of formula 2: 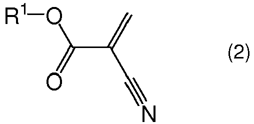 in which R¹ is alkyl, alkenyl, alkynyl, cycloalkyl or aryl, and R¹ may be optionally substituted by a halogen or a hydroxyl group.

5. Kit according to any of claims 1 to 4, **characterized in that** the cyanoacrylate (A) is selected from the group consisting of methyl 2-cyanoacrylate, ethyl 2-cyanoacrylate, propyl 2-cyanoacrylate, butyl 2-cyanoacrylate, allyl 2-cyanoacrylate, methoxyethyl 2-cyanoacrylate, ethoxyethyl 2-cyanoacrylate, 2-chloroethyl 2-cyanoacrylate, cyclohexyl 2-cyanoacrylate, ethoxycarbonylmethyl 2-cyanoacrylate, trifluoroethyl 2-cyanoacrylate and mixtures thereof.

6. Composition comprising (A) a cyanoacrylate and (B) a fluorescent agent as defined in claims 1 to 5.

7. Method of cofumigation comprising simultaneous cofumigation of a cyanoacrylate (A) and a fluorescent agent (B) as defined in claims 1 to 5 and the application of the fluorescent polymer thus formed to a surface.

8. Method according to claim 7, **characterized in that** the ratio by weight between the amount of cyanoacrylate (A) and the amount of fluorescent agent (B) is from 100:1 to 500:1, preferably from 100:1 to 200:1.

9. Method according to either of claims 7 and 8, **characterized in that** the cofumigation temperature is between 80° and 120 °C.

10. Method for visualizing traces on a substrate, comprising the steps of:
- presenting the said substrate bearing traces to a device comprising means of fumigation and containing a cyanoacrylate (A) and a fluorescent agent (B) as defined in claims 1 to 5;
- cofumigating the compounds (A) and (B) by the method as defined in claims 7 to 9 with the aid of the said device, to give a fumigated substrate;
- submitting the said fumigated substrate to ultraviolet light.

11. Use of the visualization kit according to any of claims 1 to 5 or a composition according to claim 6 for the visualization of traces on a substrate.
